# EUROPEAN PATENT SPECIFICATION

(11) **EP 4 437 984 B1**
(45) Date of publication and mention of the grant of the patent: **18.02.2026**
(21) Application number: 24167433.2
(22) Date of filing: 28.03.2024
(51) Int. Cl.: A61B 17/88

(54) **SPINE ROD CUTTER**
WIRBELSÄULENSTANGENSCHNEIDER
DISPOSITIF DE COUPE DE TIGE DE COLONNE VERTÉBRALE

(30) Priority: 31.03.2023 US 202363493579 P
(43) Date of publication of application: 02.10.2024
(73) Proprietor: Shukla Medical, St. Petersburg, FL 33709 (US)
(72) Inventor: KEACH, Nicholas Christopher, Lutz, FL 33559 (US)
(74) Representative: Keltie LLP

(56) References cited:
- US-A1- 2002 091 387
- US-A1- 2021 361 326
- US-B1- 9 427 274

## Description

### BACKGROUND OF THE INVENTION

Spinal surgery commonly entails securing spine rod implants to two or more vertebrae. The spine rod implants (or "spine rods") are secured to the vertebrae by a plurality of pedicle screws. During spine revision surgery or when simply removing the spine rods, a surgeon needs to cut the spine rod to remove the pedicle screws. During this process, the spine rod is typically cut on either side of a pedicle screw, and a screw removal tool such as a wrench or screwdriver is used to unscrew and remove the pedicle screw. A disadvantage of such a system is that it requires use of both a spine rod cutter and a separate pedicle screw removal tool in order to remove the cut spine rod and pedicle screw. That is, during an operation, the surgeon must deploy two tools to complete the job. Such a system requires an inventory of spine rod cutters and removal tools in the operating room to perform the spine rod and pedicle screw removal procedure.

Document US 2021/361326 A1 describes a cutter for cutting an implant. The cutter includes a gearbox; a primary milling bit, a secondary milling bit and a lead screw operatively coupled to the gearbox; an input coupling coupled rigidly to the primary milling bit; and an implant coupler coupled rigidly to the lead screw, wherein the gearbox is operable to translate with respect to the lead screw and to drive the secondary milling bit in response to a driving force applied to the input coupling.

### SUMMARY OF THE DISCLOSURE

According to an exemplary embodiment of the subject disclosure, there is provided a spine rod cutter including a helicopter socket comprising a substantially cylindrical body including L-shaped cut-outs for engaging a spine rod, and a hole saw coaxial with and circumscribing the helicopter socket for operatively engaging the spine rod.

According to an aspect, the spine rod cutter further comprises a driven assembly operatively engaged with the hole saw and for connecting to a drive. According to another aspect, the driven assembly comprises a drive connector and a thrust housing for operatively engaging the hole saw.

According to an aspect, the spine rod cutter further comprises a guard covering the hole saw. According to another aspect, the spine rod cutter further comprises a centering mount within the helicopter socket. According to another aspect, the spine rod cutter further comprises a handle having a lever operatively connected to the hole saw for applying an axial load to the hole saw. According to another aspect, the spine rod cutter further comprises an engagement assembly releasably engaging the hole saw and helicopter socket to operate cooperatively.

According to an aspect, the spine rod cutter further comprises a hole saw extension extending from the hole saw, and a helicopter socket extension extending from the helicopter socket, wherein the engagement assembly operatively connects the hole saw extension and helicopter socket extension together. According to another aspect, the engagement assembly includes a knob circumscribing the helicopter socket extension, a sleeve extending from the hole saw extension, a transfer housing engaged with the helicopter socket extension, and a fastener for operatively engaging both the sleeve and transfer housing.

According to an aspect, the helicopter socket extension includes a colleted proximal end, and the driven assembly comprises a drive connector, a thrust pin housed within the drive connector operatively engaging the proximal end of the helicopter socket extension, a thrust housing for operatively engaging the hole saw, and a thrust race between the thrust housing and the helicopter socket extension. According to another aspect, the driven assembly further comprises a sleeve lock connected to the thrust pin for moving the thrust pin between first and second positions.

According to another aspect, the guard circumscribes the helicopter socket and the hole saw.

Other features and advantages of the subject disclosure will be apparent from the following more detailed description of the exemplary embodiments.

### BRIEF DESCRIPTION OF THE SEVERAL VIEWS OF THE DRAWINGS

The foregoing summary, as well as the following detailed description of the exemplary embodiments of the subject disclosure, will be better understood when read in conjunction with the appended drawings. For the purpose of illustrating the present disclosure, there are shown in the drawings exemplary embodiments. It should be understood, however, that the subject application is not limited to the precise arrangements and instrumentalities shown.
FIG. 1 is a perspective view of a spine rod cutter in accordance with an exemplary embodiment of the subject disclosure;
FIG. 2 is an elevational cross-sectional view of a spine rod cutter in accordance with an exemplary embodiment of the subject disclosure;
FIG. 3 is a top perspective view of a drive connector of the spine rod cutter of FIG. 1;
FIG. 4 is a bottom perspective view of the drive connector of FIG. 3;
FIG. 5 is a top perspective view of a sleeve lock of the spine rod cutter of FIG. 1;
FIG. 6 is a bottom perspective view of the sleeve lock of FIG. 5;
FIG. 7 is a top perspective view of a thrust housing of the spine rod cutter of FIG. 1;
FIG. 8 is a bottom perspective view of the thrust housing of FIG. 7;
FIG. 9 is a top perspective view of a sleeve of the spine rod cutter of FIG. 1;
FIG. 10 is a bottom perspective view of the sleeve of FIG. 9;
FIG. 11 is a top perspective view of a knob of the spine rod cutter of FIG. 1;
FIG. 12 is a bottom perspective view of the knob of FIG. 11;
FIG. 13A is an elevational view of a portion of an engagement assembly of the spine rod cutter of FIG. 1;
FIG. 13B is another elevational view of a portion of an engagement assembly of the spine rod cutter of FIG. 1;
FIG. 13C is another elevational view of a portion of an engagement assembly of the spine rod cutter of FIG. 1
FIG. 14 is a top perspective view of a handle of the spine rod cutter of FIG. 1;
FIG. 15 is a bottom perspective view of the handle of FIG. 14;
FIG. 16 is a top perspective view of a lever of the spine rod cutter of FIG. 1;
FIG. 17 is a perspective view of the lever of FIG. 16;
FIG. 18 is a top perspective view of a sliding sleeve of the spine rod cutter of FIG. 1;
FIG. 19 is a bottom perspective view of the sliding sleeve of FIG. 18;
FIG. 20 is a top perspective view of a hole saw extension of the spine rod cutter of FIG. 1;
FIG. 21 is a bottom perspective view of the hole saw extension of FIG. 20;
FIG. 22 is a top perspective view of a hole saw of the spine rod cutter of FIG. 1;
FIG. 23 is a bottom perspective view of the hole saw of FIG. 22;
FIG. 24 is an elevational view of the hole saw of FIG. 22;
FIG. 25 is a top perspective view of a helicopter socket of the spine rod cutter of FIG. 1;
FIG. 26 is a bottom perspective view of the helicopter socket of FIG. 25;
FIG. 27 is a bottom view of the helicopter socket of FIG. 25;
FIG. 28 is a front elevational view of a centering mount of the spine rod cutter of FIG. 1;
FIG. 29 is a front elevational view of a helicopter socket extension of the spine rod cutter of FIG. 1;
FIG. 30 is a top perspective view of the helicopter socket extension of FIG. 29;
FIG. 31 is a bottom perspective view of the helicopter socket extension of FIG. 29;
FIG. 32 is an elevational cross-section view of a thrust race of the spine rod cutter of FIG. 1;
FIG. 33 is a front view of a thrust pin of the spine rod cutter of FIG. 1;
FIG. 34 is a top perspective view of a guard of the spine rod cutter of FIG. 1;
FIG. 35 is a bottom perspective view of the guard of FIG. 34;
FIG. 36 is an elevational view of a transfer housing of the spine rod cutter of FIG. 1;
FIG. 37 is a top perspective view of the transfer housing of FIG. 36; and
FIG. 38 is a plan view of a conventional spine rod implant connected to a portion of a spine.

### DETAILED DESCRIPTION

Reference will now be made in detail to the various exemplary embodiments of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1 %, or ±0.1% from the specified value, as such variations are appropriate.

"Substantially" as used herein shall mean considerable in extent, largely but not wholly that which is specified, or an appropriate variation therefrom as is acceptable within the field of art. "Exemplary" as used herein shall mean serving as an example.

Throughout this disclosure, various aspects of the subject disclosure can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments. One skilled in the relevant art will recognize, in light of the description herein, that the present disclosure can be practiced without one or more of the specific features or advantages of a particular exemplary embodiment. In other instances, additional features and advantages may be recognized in certain embodiments that may not be present in all exemplary embodiments of the subject disclosure.

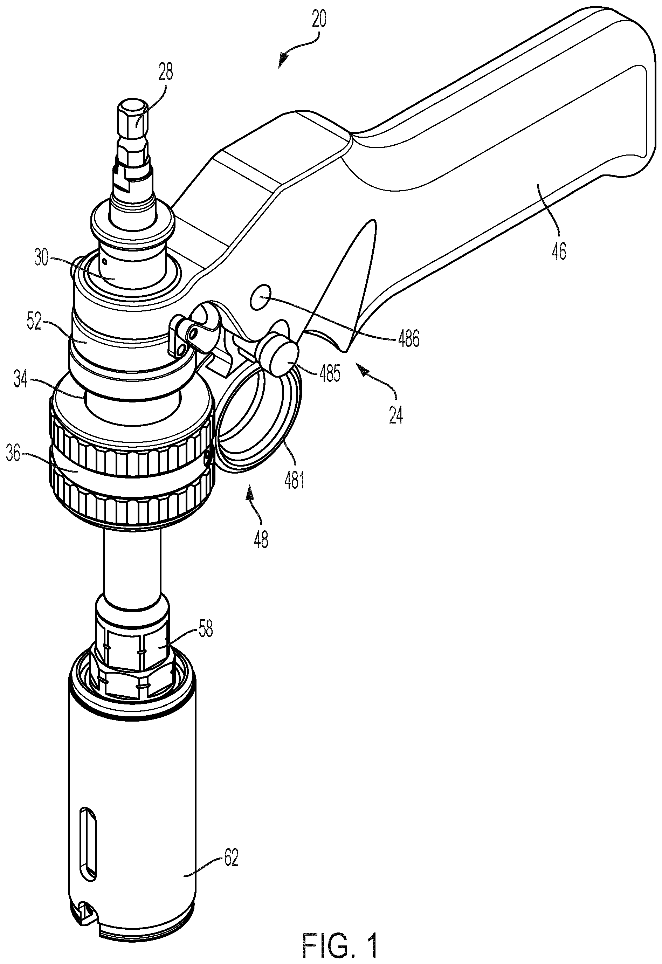

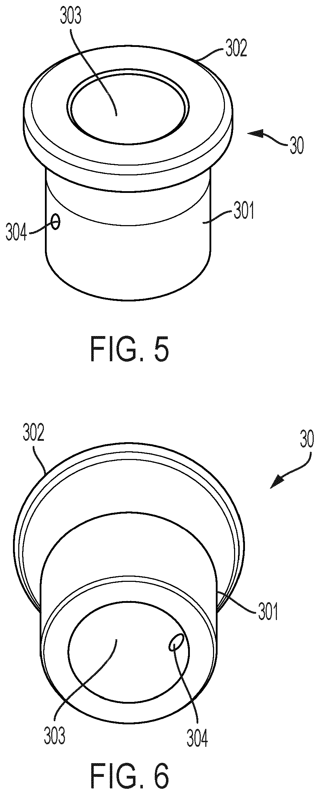

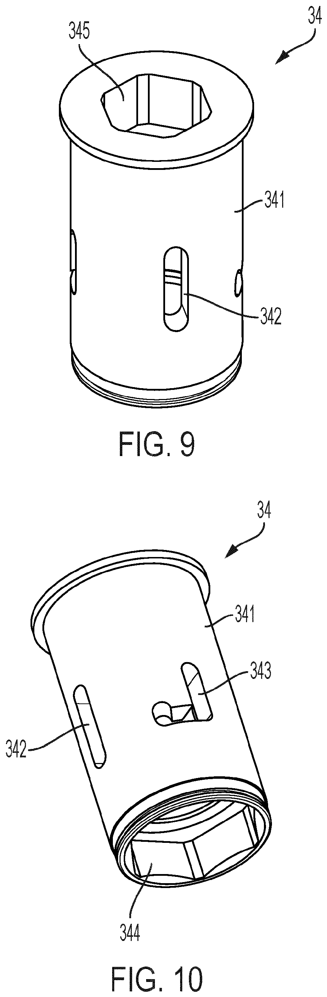

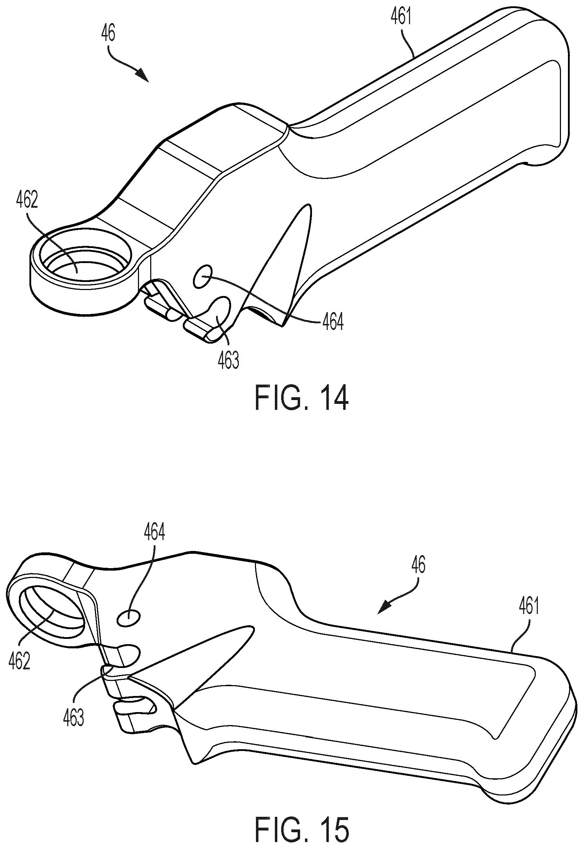

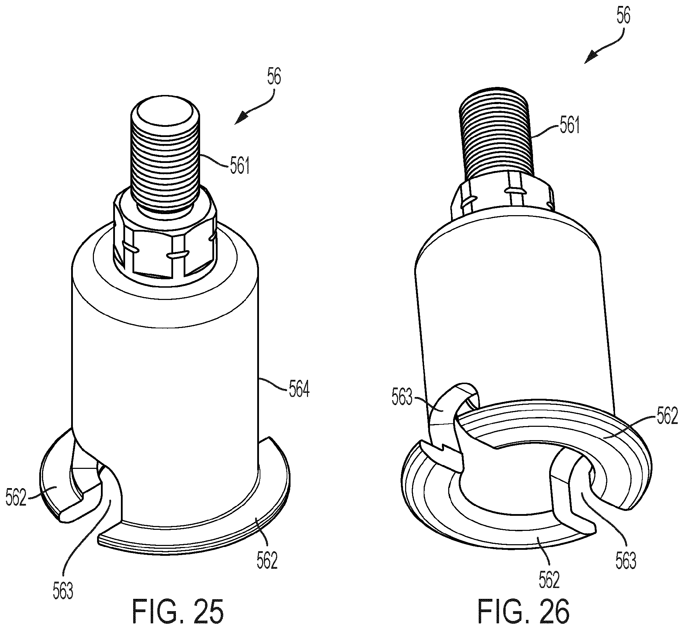

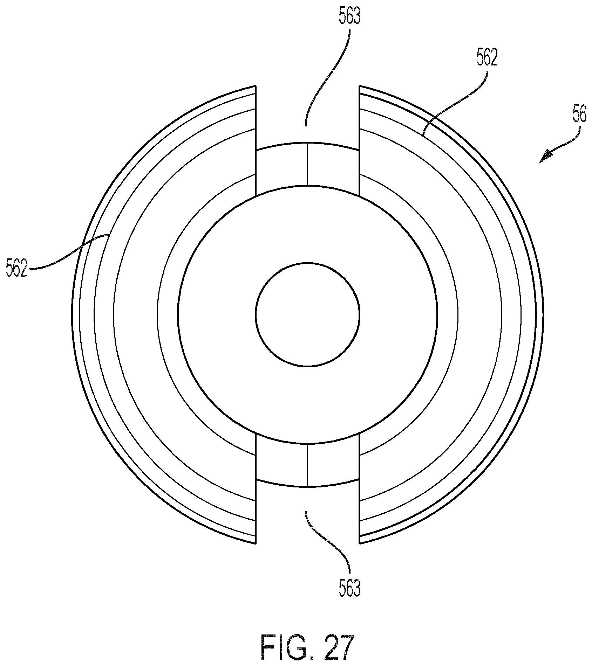

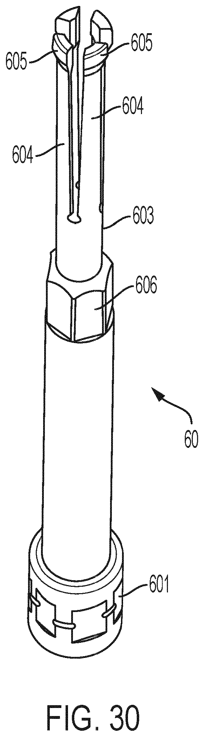

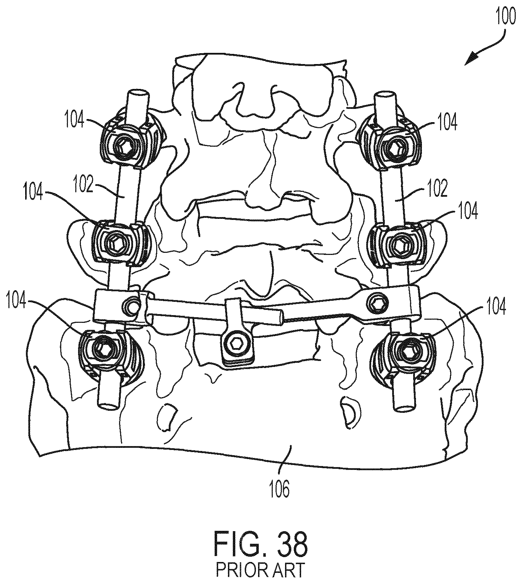

Referring now to the examples shown in FIGS. 1-38, the subject disclosure provides for a spine rod cutter and pedicle screw remover or, simply, spine rod cutter 20. The spine rod cutter 20 is used for removal of previously implanted spinal implants 100, and specifically for removal of pedicle screws and associated spine rods. In some examples, such as the example shown in FIG. 38, the implant 100 is a spinal fusion implant that includes at least one spine rod 102 and one or more pedicle screws 104 for securing the rod to bone 106, e.g., a plurality of vertebrae. The spine rod cutter 20 is configured for cutting an elongate section of the rod 102 that is implanted within a patient and removing the pedicle screws 104 following cutting of the spine rods.

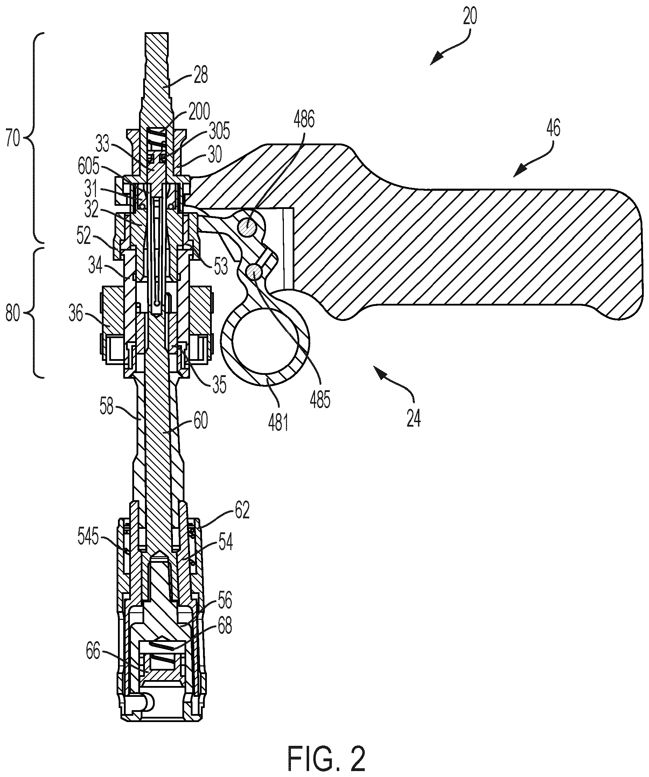

According to the subject disclosure and as shown in FIG. 2, the spine rod cutter 20 includes a helicopter socket 56 for engaging a spine rod and a hole saw 54 coaxial with the helicopter socket for operatively engaging the spine rod. The spine rod cutter further comprises a driven assembly 70 operatively engaged with the hole saw 54 and for connecting to a drive (not shown), e.g., a reversible rotary drive source. The driven assembly 70 comprises a drive connector 28 and a thrust housing 32 for operatively engaging the hole saw 54, and more specifically, a hole saw extension 58, as described below. The spine rod cutter 20 further includes an engagement assembly 80 releasably engaging the hole saw 54 and the helicopter socket 56 to operate cooperatively. More particularly, the engagement assembly 80 operatively connects the hole saw extension 58 and a helicopter socket extension 60 together, as further described below. The spine rod cutter 20 further includes a handle 46 having a lever 48 pivotably connected to the handle and operatively connected to the hole saw for applying an axial load to the hole saw 54, as described below. Additionally, the spine rod cutter includes a knob 36 for selectively coupling the driven assembly 70 and the engagement assembly 80.

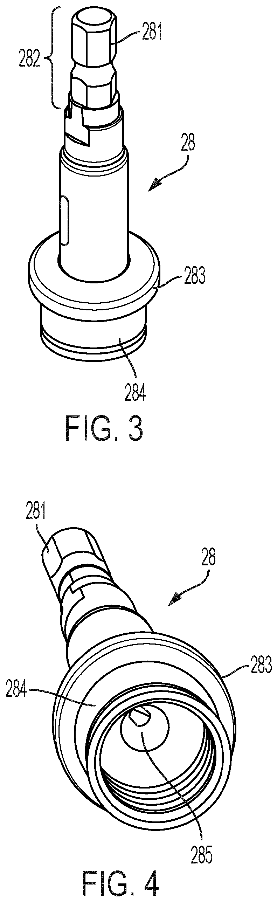

Referring to FIGS. 1 and 2, the driven assembly 70 is configured to translate power to the hole saw. The driven assembly includes a drive connector 28, a sleeve lock, a thrust pin, and a thrust housing. The drive connector 28 is configured as best shown in FIGS. 1, 3 and 4 and is operable to couple to a reversible rotary power source (not shown), e.g., a hand-operated drill or the like, configured to provide reversible rotational motion to the hole saw 54. In an exemplary embodiment, the drive connector 28 includes at its proximal end at least one longitudinally extending planar surface 281 configured to be engaged by cooperating longitudinally extending planar surface(s) of the power source such that the power source can translate rotational motion to the drive connector. Referring to FIG. 3, in accordance with an exemplary embodiment, a proximal end of the drive connector is structured as a quick connection 282, e.g., a male quick connection, for easy connection to and disconnection from the power source. The drive connector further includes a radially outwardly extending flange 283 and an internally threaded distal cylindrical portion 284, the functions of which are described below.

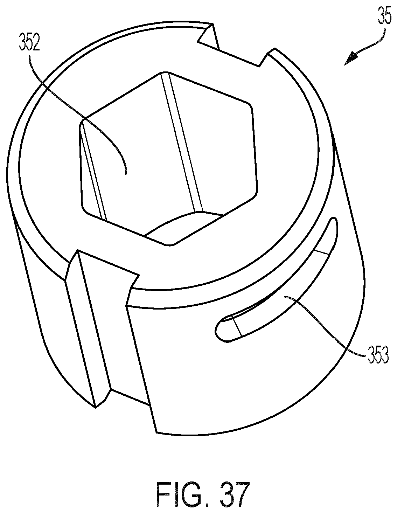

A sleeve lock 30 is configured as best shown in FIGS. 1, 5 and 6 and has a hollow cylindrical body 301 bounded on a proximal end by a radially outwardly extending flange 302. The sleeve lock includes a central through opening 303 having a diameter slightly larger than the distal cylindrical portion 284 of the drive connector 28 such that the sleeve lock through opening 303 receives the distal cylindrical portion of the drive connector and the sleeve lock flange 302 becomes seated against the flange 283 of the drill connecter (see FIG. 1). The sleeve lock 30 further includes at least one radially directed aperture 304 extending through the hollow cylindrical body 301 to allow a fastener e.g., a pin 305(FIG. 2) to secure the sleeve lock 30 to the driven assembly. The sleeve lock is operatively connected to a thrust pin 33 (FIG. 37, and further described below) for moving the thrust pin between first and second positions, whereby the sleeve lock is operable to be raised and lowered along the drive connector 28 in order to permit assembly and disassembly of the spine rod cutter 20. The sleeve lock is connected to the thrust pin via pin 305 (as shown in FIG. 2)

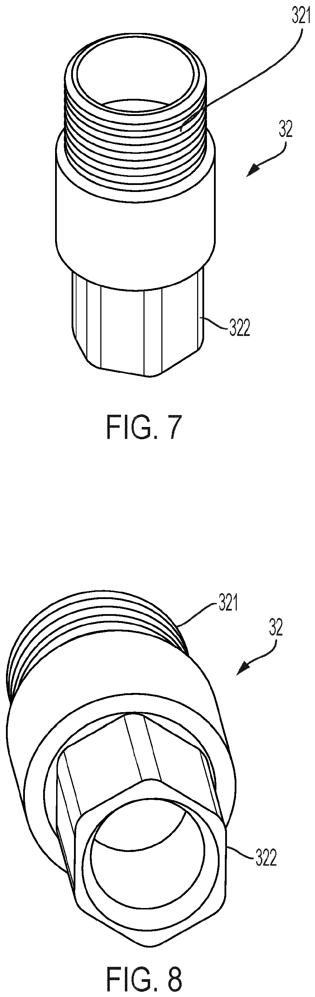

The thrust housing 32 is configured as best shown in FIGS. 7 and 8 and enables rotational movement to be transferred from the drive connector 28 to the remaining components of the spine rod cutter. The thrust housing 32 includes an externally threaded cylindrical portion 321 about its proximal end and a connector portion 322 about its distal end. The connector portion 322 can be a polygonal shaped male connector portion, such as a hexagonal shaped male connector portion. However, the proximal end and distal end can alternatively be configured with other configurations and/or shapes or transverse cross-sections suitable for their intended purposes. The externally threaded cylindrical portion 321 is configured to be threadedly received within the internally threaded distal cylindrical portion 284 of the drive connector 28 to establish a thread lock therewith. In other words, once assembled, the drive connector 28 is affixed or locked to the thrust housing 32. The connector portion 322 is configured to slidingly engage with the sleeve 34, as further discussed below

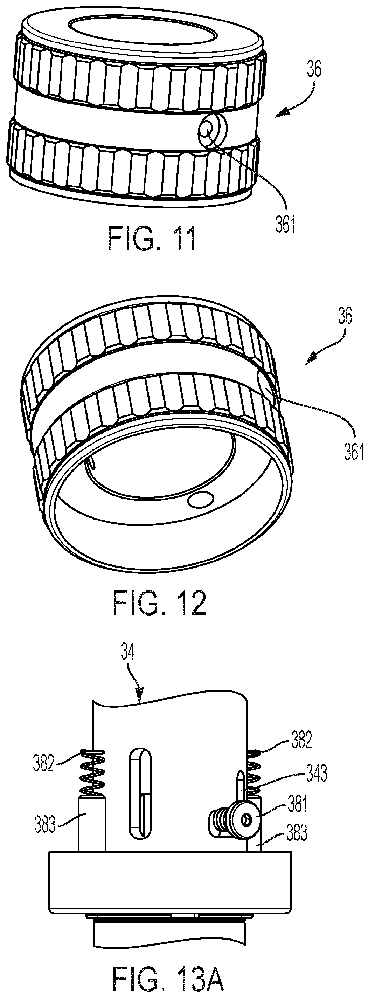

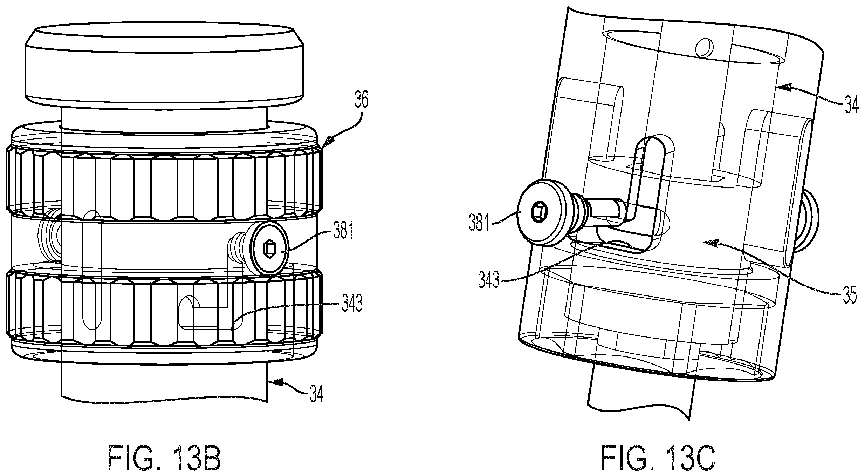

The engagement assembly 80 is configured as best shown in FIGS. 1, 2, 11, 12 and13A-13C. The engagement assembly includes the knob 36, which is configured as best shown in FIGS. 1, 11 and 12. The knob has a generally cylindrical body having a plurality of recessed exterior portions for providing a grip-enhancing surface for user engagement thereof, as well as a radial opening 361 in a side wall thereof for receiving a fastener 381 (FIG. 13) such as a set screw, a pin or the like. The knob 36 is movable both axially and rotationally with respect to the sleeve 34. As shown in FIG. 2, the knob 36 circumscribes the helicopter socket extension 60 and the sleeve.

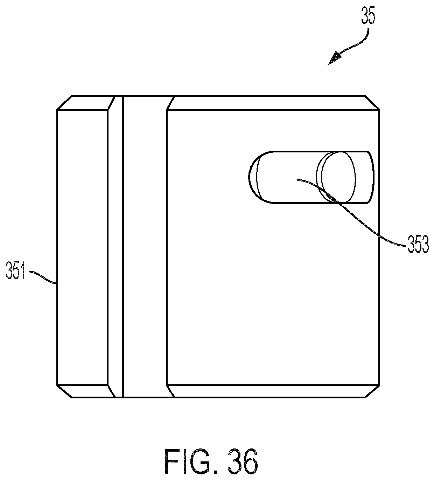

The engagement assembly 80 further includes a transfer housing 35 operatively engaged with the helicopter socket extension 60. The transfer housing is configured as best shown in FIGS. 36 and 37 and includes a substantially cylindrical body 351 with a keyed central through hole 352, such as a polygonal keyed through hole e.g., a hexagonal shaped central through hole. The keyed central through hole is shaped complimentary to the helicopter socket extension proximal end so as to slidingly engage therewith. The transfer housing also includes a horizontal slot 352 for receiving the fastener 381, as described below.

, The engagement assembly operates to releasably engage the hole saw and helicopter socket such that they operate cooperatively. That is, the knob 36 is configured to actuate the transfer housing 35 and engage it with the sleeve 34 such that the sleeve and transfer housing move cooperatively e.g., rotation of the sleeve drives rotation of the transfer housing which in turn drives rotation of the helicopter socket via the helicopter socket extension 60. The sleeve 34 is operatively connected to the transfer housing via set screw 381, as shown in FIGS. 13B and 13C.

The sleeve 34 is configured as best shown in FIGS. 2, 9 and 10 and includes a generally cylindrical body 341 having an outer surface to allow the knob 36 to slide relative thereto. The sleeve 34 may also define one or more linear slots 342 and one or more L-shaped slots 343 circumferentially spaced about the generally cylindrical body. The slots 342, 343 are configured to receive set screws or pins 381 to connect the sleeve to the transfer housing. For example, the L-shaped slots 343 are configured to allow movement of the knob 36 between first and second axial positions and a rotated position and a non-rotated position, as further described below. A distal portion of the sleeve 34 may include a polygonal female socket 344 comprising a plurality of planar surfaces configured to be coupled to a plurality of external planar surfaces 582 of the hole saw extension 58. That is, the polygonal female socket 344 is configured to be complementarily shaped with the proximal external surfaces of the hole saw extension. Moreover, a proximal portion of the sleeve 34 may also include an additional polygonal female socket 345 comprising a plurality of planar surfaces configured to be coupled to a plurality of planar surfaces of another component, namely, the polygonal shaped portion 322 at the distal end of the thrust housing 32. That is, the proximal portion of the sleeve is configured to have a polygonal female socket shaped complementarily to the shape of the distal end of the thrust housing.

As best shown in FIGS. 13A-13C, the engagement assembly 80 is configured to secure the knob 36 in desired axial and angular (rotated) positions. The locking mechanism 38 includes the fastener 381, at least one biasing member 382 biasing at least one dowel pin 383 extending from a slot 342 in the sleeve 34, thus biasing the knob 36 upwardly relative to the sleeve. The fastener 381 is configured to be disposed in the radial opening 361 of the knob 36, through the L-shaped slot 343 in the sleeve 34 to secure the knob 36 in a rotated position or in the non-rotated position. In the rotated position, the fastener 381 resides in a horizontal leg of the L-shaped slot 343 of the sleeve 34 and a horizontal slot 351 in the transfer housing 35 (FIGS. 36 and 37). In order to move axially in relation to the sleeve 34, the knob 36 is rotated to along the horizontal leg of the L-shaped slot 343 until the axial leg of the L-shaped slot is reached. Upon reaching the axial leg of the L-shaped slot, the knob 36 is lifted by biasing member(s) 382 along the axial leg of the L-shaped slot until a polygonal, e.g., hex-shaped, opening 352 (FIG. 37) is disengaged from the polygonal formation 606 provided on the helicopter socket extension 60. It is understood that the reverse operation engages the polygonal opening 352 of the transfer housing 35 with the polygonal formation 606 provided on the helicopter socket extension 60, whereby the hole saw and the helicopter socket are selectively engaged and disengaged to operate cooperatively. Moreover, the dowel pin(s) 383 are configured to prevent undesirable lateral movement of the knob 36 during manipulation thereof by a user. In some examples, the knob 36 is configured to rotate approximately 20-40 degrees, including 15, 25, 30, 35 and 45 degrees; however, various other rotational configurations have been contemplated.

When assembled, the drive connector 28 is disposed about a proximal end of the spine rod cutter 20 (as shown in FIG. 2) with the sleeve lock 30 circumscribing the drive connector 28 and being disposed adjacent to the drive connector's distal end. The thrust housing 32 is coupled to the drive connector 28 and is disposed distally adjacent thereto. The thrust housing 32 is rigidly locked to the drive connector via a cooperating thread lock on the drive connector and thrust housing. The sleeve 34 is slidingly coupled to the thrust housing via its male and female keyed coupling and disposed distally adjacent to the thrust housing. The knob 36 at least partially circumscribes the sleeve 34 and the transfer housing and is configured to rotatably and axially slide about or along the sleeve.

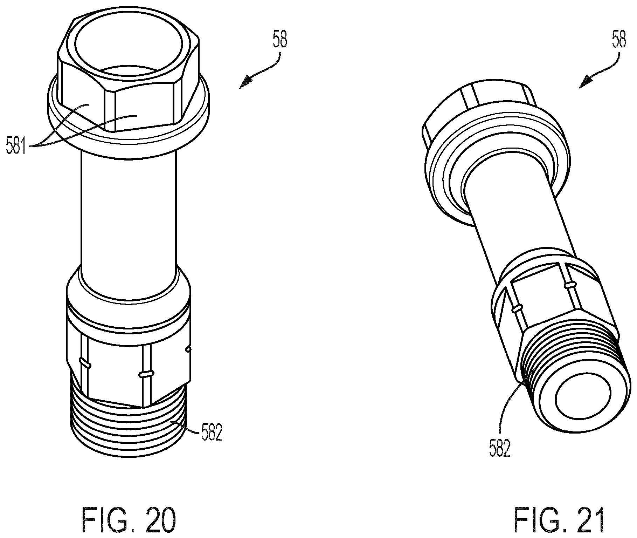

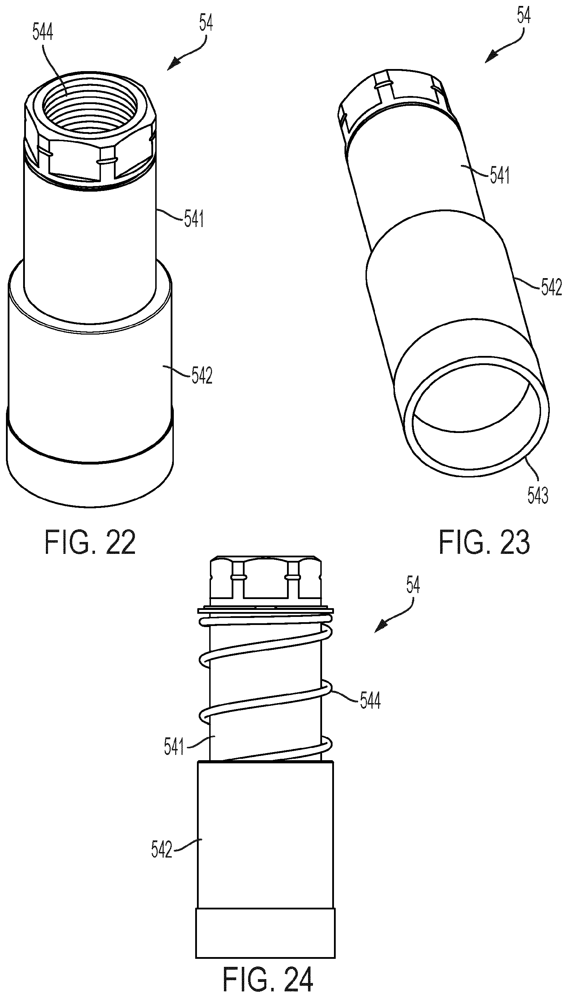

In accordance with an exemplary embodiment, the hole saw extension 58 is configured as shown in FIGS. 20 and 21. The hole saw extension 58 is attached to and extends from the hole saw 54 and is configured to transmit power from the power source thereto. To facilitate this, the hole saw extension 58 includes a plurality of planar surfaces 581 disposed about its proximal end to facilitate coupling of the hole saw extension to socket 344 of the sleeve 34. Additionally, the hole saw extension 58 includes an externally threaded distal end 582 for threadedly connecting to an internally threaded proximal end 544 of the hole saw 54 (FIG. 22). The hole saw extension 58 may be various lengths including but not limited to 3, 4, 5, 6, 7, 8, 9 or more inches to increase or decrease overall rod cutter length as needed.

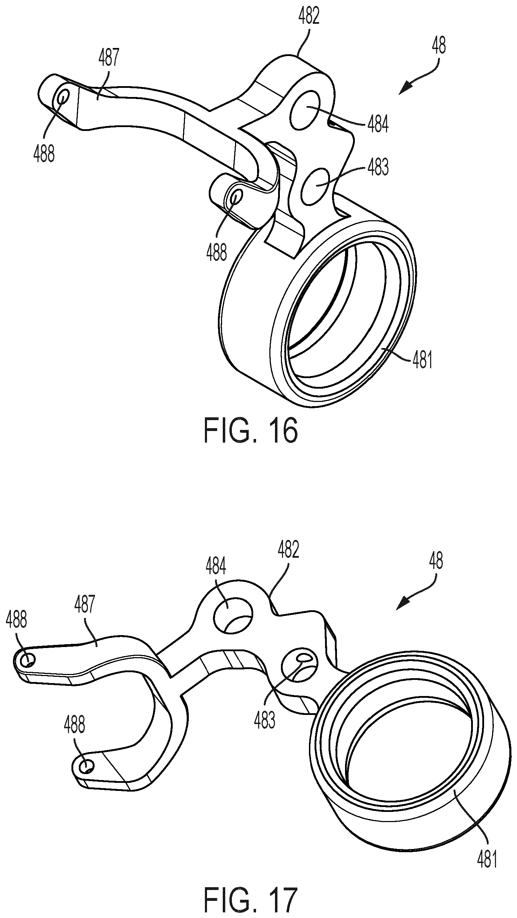

In accordance with an exemplary embodiment, the spine rod cutter also includes a handle assembly 24 having a handle 46 (FIGS. 2, 14 and 15) and a lever 48 (FIGS. 16 and 17). The handle 46 is configured to be engaged by a hand of a user and is sized and shaped for that purpose. In the example shown, the handle 46 is solid and includes a grip 461 at a first end thereof which is ergonomically shaped for a user to easily engage the handle with a single hand. A second end of the handle includes an opening 462 for receiving the distal end 284 of the drive connector 28 and the proximal end 321 of the thrust housing 32. Between the first and second ends of the handle there is provided a bifurcated recess 463 and a pair of aligned openings 464 (only one of which is shown) for connecting the lever 48 to the handle 46.

As shown in FIGS. 16 and 17 a first end of the lever 48 includes a finger-operated trigger 481 joined to a central region 482 having first and second through holes 483, 484 for securing the trigger assembly to the bifurcated recess 463 and the aligned openings 464 of the handle 46 via pins 485, 486 or the like (FIGS. 1 and 2). A second end of the lever includes a generally Y-shaped coupler 487. The trigger 481 is generally circular and configured to allow at least one of a user's fingers to be inserted therethrough. Further, the trigger 481 is configured to be pivotably movable by a user's finger about pin 486 between a disengaged position and an engaged position. The free ends of opposed arms of the generally Y-shaped coupler 487 include openings 488 for receiving pins or the like (not shown) for connecting the trigger assembly 48 to a sliding sleeve 52, further discussed below. The generally Y-shaped coupler 487 is configured to translate the force from a user pulling the trigger 481 into axial movement of the sliding sleeve 52 thereby applying a distally directed force of the hole saw on the spine rod to be cut.

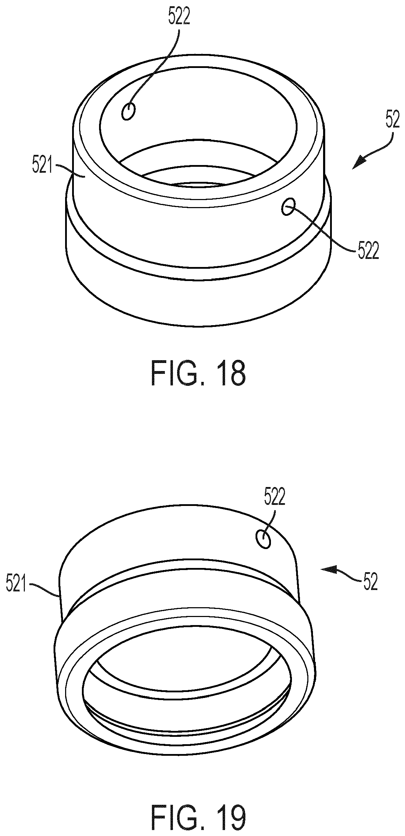

The sliding sleeve 52 is generally configured as shown in FIGS. 18 and 19. The sliding sleeve has a generally cylindrical body 521 including a pair of radial openings 522 that are coupled to the openings 488 in the opposed arms of the Y-shaped coupler 487 by the aforementioned pins. The sliding sleeve is also configured to cooperate with a flanged bushing 53 which engages an underside of a flange extending from the sleeve 34 so as to operatively connect the sleeve 34 to the sliding sleeve. So arranged, the sliding sleeve 52 is disposed co-axial with the thrust housing 32.

The sliding sleeve 52 is coupled to the trigger 481 such that movement of the trigger between a disengaged position and an engaged position moves the sliding sleeve 52 between a proximal position at which no axial load is applied to the hole saw 54 and a distal position whereupon an axial load is applied to the hole saw 54 for cutting through a spine rod. In other words, the trigger allows a user to move the sliding sleeve up and down to apply pressure on the hole saw as desired.

In accordance with an exemplary embodiment, the hole saw 54 is configured as best shown in FIGS 22-24. The hole saw 54 has a generally cylindrical body and configured to rotate along its central longitudinal axis in either rotational direction and configured to circumscribe the helicopter socket 56. The hole saw 54 includes an upper cylindrical body 541 having a smaller diameter than a lower cylindrical body 542. The lower cylindrical body includes a distally-facing annular cutting surface 543. In some examples, the cutting surface 543 includes a diamond grit coating; however, various other cutting surface materials and/or shapes suitable for its intended purpose are contemplated. The hole saw further includes an upper or proximal internally threaded proximal end 544 for threadedly connecting the hole saw 54 to the externally threaded distal end 582 of the hole saw extension 58. The hole saw has an inner diameter sufficient to receive the helicopter socket therein e.g., 0.5, 1, 1.5, 2 or more inches. The upper cylindrical body 541 may be circumscribed by a biasing member 545 such as a compression spring, an elastomer, or the like, which is configured to bias a guard assembly 62 into contact with a spine rod to be cut, as further described below and as shown in FIG. 2. In operation, the annular hole saw cuts the spine rod 102 on both sides the pedicle screw 104 simultaneously while keeping a neutral force on the remaining portions of the rod.

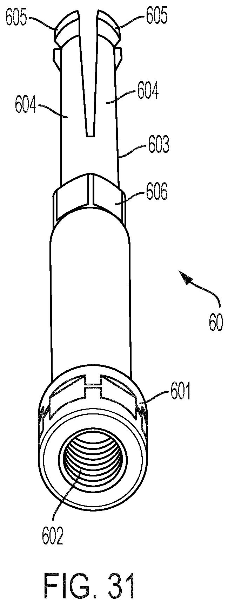

In accordance with an exemplary embodiment, the helicopter socket 56 is configured as best shown in FIGS. 1, 2 and 25-27 and is received within the hole saw 54. The helicopter socker 56 has proximal connection end in the form of an externally threaded proximal end 561 configured to threadedly engage the internally threaded distal socket 602 of the helicopter socket extension 60 (FIG. 31) to facilitate coupling therewith and a substantially cylindrical body 564. A flange 562 extends radially outwardly from and is disposed about a distal end of the helicopter socket body. When assembled with the hole saw, the flange 562 is disposed distally to the hole saw 54 such that the hole saw may engage a top surface of the flange while the flange prevents undesirable cutting of soft tissue below. The flange 562 is generally circular to mirror the cylindrical saw shape. Further, the flange 562 and a distal portion of the helicopter socket body includes L-shaped cut-outs 563 to accommodate and stabilize the spine rod 102 therein during cutting. In some examples, the spine rod cutter may be fitted with one of a plurality of helicopter sockets 56 configured to have various extension lengths and the plurality of helicopter sockets 56 are interchangeable with one another based on the depth or type of implant 100 to be removed.

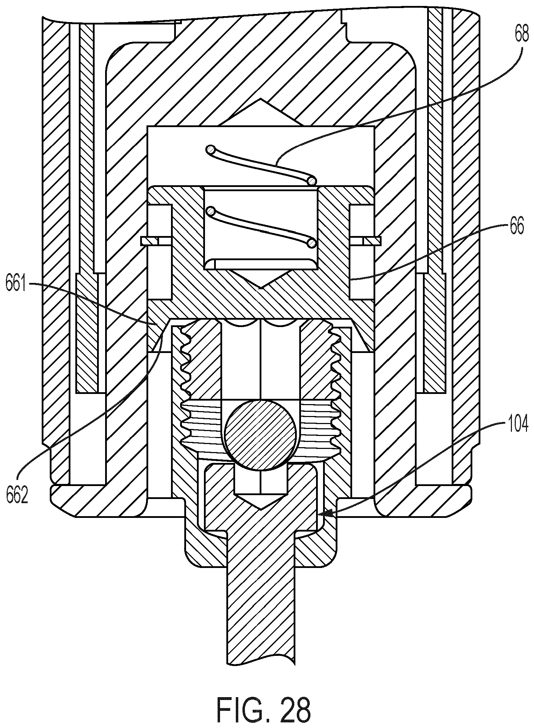

In accordance with an exemplary embodiment, the spine rod cutter includes a centering mount 66 configured as best shown in FIG. 28 and is mounted within the helicopter socket 56 and configured to center the cutter 20 about the pedicle screw 104. The centering mount 66 is configured to ensure rod 102 is cut evenly on both sides of the pedicle screw 104. To facilitate this, a distal end 661 of the centering mount has an inwardly tapered portion 662 which is configured to engage the head or tulip of a pedicle screw 104 and assist in centering the pedicle screw relative to the hole saw 54. The centering mount 66 is also coupled to a biasing member 68, e.g., a compression spring, an elastomer, or the like, the opposite end of which contacts the helicopter socket (FIG. 2). The biasing member 68 biases the centering mount in a distal direction such that the centering mount may contact the pedicle screw 104 when desired. As will be described in greater detail below, the centering mount 66 and the biasing member 68 provide a clamping force to firmly engage the spine rod cutter 20 with the pedicle screw 104 in order to remove the pedicle screw following cutting of the spine rod.

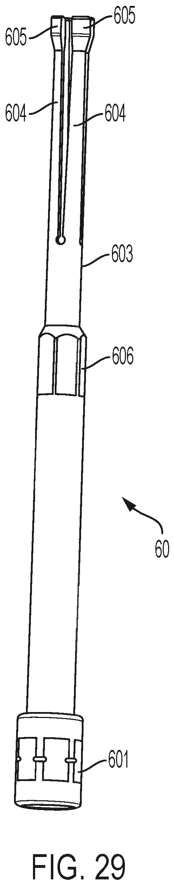

In accordance with an exemplary embodiment, the helicopter socket extension 60 is configured as best shown in FIGS. 29-31. The helicopter socket extension 60 extends proximally from the helicopter socket 56 and extends through the hole saw extension 58, the sleeve 34, and the thrust housing 32. The helicopter socket extension 60 may be any sized diameter corresponding with any sized hole saw extension 58. The helicopter socket extension 60 is an elongated member having a distal end 601 which defines an internally threaded socket 602 configured to receive the externally threaded proximal end 561 of the helicopter socket 56. A proximal end 603 of the helicopter socket extension is colleted or configured as a plurality of collet fingers 604. In the illustrated example, there are four such collet fingers, although there can be as few as two or more than four. The free ends of the collet fingers are provided with radially outwardly directed tabs 605 which are received in an annular thrust race 31, as shown in FIG. 2. As noted above, the helicopter socket extension further includes a polygonal formation 606 provided generally at a mid-region thereof which is adapted for selectively engaging and disengaging a polygonal opening 352 (FIG. 37) of the transfer housing 35. The helicopter socket extension 60 may be various lengths corresponding to the length of the hole saw extension 58 to increase or decrease overall rod cutter length as needed.

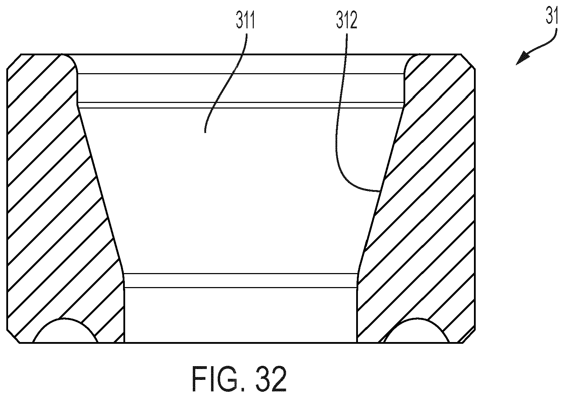

The thrust race 31 is shown in cross-section in in FIG. 32 and is sized to be seated in the generally cylindrical portion 321 of the thrust housing 32 which operatively engages the hole saw. The thrust race is disposed between the thrust housing and the helicopter socket extension 60. The thrust race includes an axial through bore 311 having a first diameter at a proximal end and a second smaller diameter at a distal end, wherein the proximal and distal ends of the bore are joined by a continuously tapered inner wall 312. The tapered inner wall is sized such that it can accommodate the outwardly directed tabs 605 of the collet fingers 604 in a fully expanded state (FIG. 2). Additionally, the distal second diameter of the through bore 311 is sized such that it can permit passage therethrough of the outwardly directed tabs 605 of the collet fingers 604 in a contracted state to facilitate assembly of the helicopter socket extension 60 to the thrust race 31.

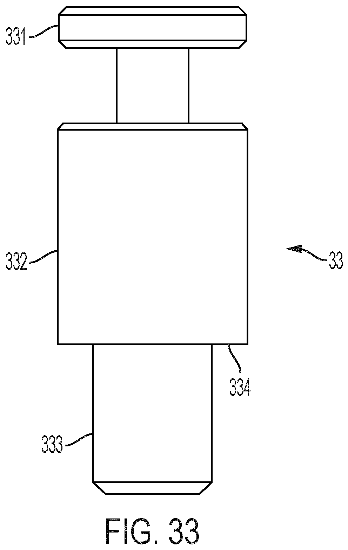

FIG. 33 illustrates additional structures of the driven assembly 70, namely, a biased thrust pin 33 which is housed within the drive connector 28 and operatively engages the proximal (colleted) end of the helicopter socket extension 60. More particularly, the thrust pin 33 is located proximal to the annular race 31, as shown in FIG. 2. The thrust pin has a proximal end 331 that is adapted to be contacted by a first end of a biasing member 200 (FIG. 2) such as a compression spring, an elastomer, or the like. The second end of the biasing member 200 contacts a base of a socket 285 (FIG. 4) provided in the drive connector 28. The thrust pin 33 further includes a central portion 332 and a distal portion 333. The distal portion 333 has a smaller diameter than the central portion 332 so as to define a distally facing annular shoulder 334 therebetween. As best shown in FIG. 2, the diameter of the distal portion is such that the distal portion fits within the collet fingers of the helicopter socket extension 60 and firmly holds the outwardly directed tabs 605 of the collet fingers into contact with the tapered inner wall of the thrust race.

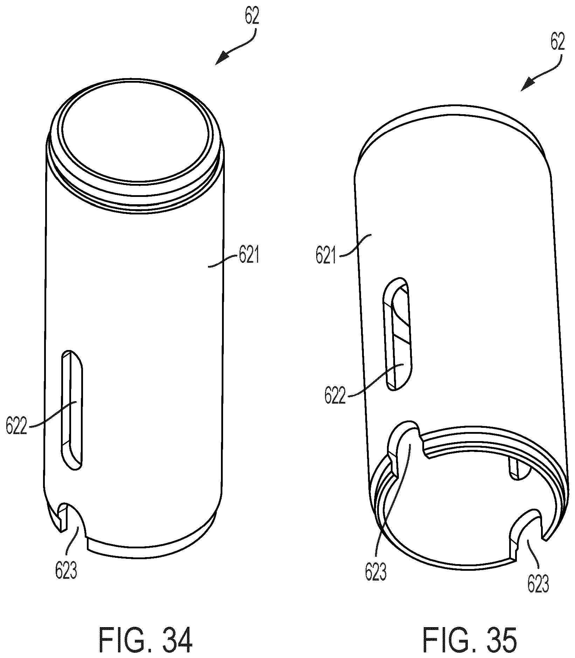

In accordance with an exemplary embodiment, the guard assembly 62 is configured as best shown in FIGS. 34 and 35 and is operable to protect the hole saw 54 from interference and debris. According to the subject disclosure, the guard assembly 62 includes a generally cylindrical guard 621 covering the hole saw 54 and circumscribing the helicopter socket 56 and the hole saw. In some examples, the guard 621 can be formed out of a clear material, e.g., a clear polymer such as polycarbonate, to provide a barrier between the hole saw 54 and the user and to enable the user to see rod cuttings as they are cut by the hole saw. The guard 621 may also define at least one coolant slot 622 for allowing fluid to pass therethrough. The fluid may be water, saline, or another coolant configured to enter through the coolant slot 622 to resist heat build-up during operation. Additionally, the guard assembly includes opposed cut-outs 623 at its distal end which align with the cut-outs 563 of the helicopter socket 56.

With the spine rod cutter fully assembled as shown in FIGS. 1 and 2, the user rotates the knob 36 from the rotated to the non-rotated position whereby the distal end 322 of the thrust housing 32 disengages from the socket 345 at the proximal end of the sleeve under the influence of the biasing member(s) 382 located within the knob. Thereafter, the drive connector 28 of the spine rod cutter 20 may be coupled to the reversible rotary power source and the helicopter socket 56 can be placed in engagement with the rod 102 of the implant 100 circumscribing the pedicle screw 104. The centering device 66 of the helicopter socket 56 is placed on the pedicle screw 104 with the rod 102 disposed within the cut outs of the flange 64. When downward pressure is applied, the biasing device 68 of the centering device 66 provides a clamp force to the rod 102 to secure the rod 102 thereon. The user then grips the handle 46 and inserts his or her finger into the trigger 481. To cut the rod, the rotary power source is activated and the user squeezes the trigger 481 which presses the sliding sleeve 52 distally into contact with the sleeve 34 to apply an axial load to and move the hole saw 54 in an distal axial direction. Rotational movement from the power source in a first direction is translated from the drive connector 28, to the thrust housing 32, to the sleeve 34, to the hole saw extension 58, and finally to the hole saw 54. The hole saw 54 then rotates about the pedicle screw 104 to cut the rod 102 of the implant 100. Once the rod is cut, the user removes his or her finger from the trigger 481, the polygonal shaped distal end 322 of the thrust housing 32 disengages from the socket 345 of the sleeve 34 whereby rotation of the hole saw 54 stops.

The user may then deactivate the rotary power source and slide the knob 36 distally or downwardly and rotate it to the rotated position which locks the helicopter socket 56 to the hole saw 54, as described above. The user then squeezes the trigger 481 to engage the polygonal shaped distal end 322 of the thrust housing 32 with the socket 345 of the sleeve 34. Thereafter, the user activates the rotary power source to rotate in the opposite direction and back the pedicle screw 104 out and remove it from the patient. In the alternative, with the helicopter socket locked to the hole saw, the user can connect a T-handle to the drive connector 28 and unscrew the pedicle screw from the patient.

The modular design of the spine rod cutter 20 allows for various sizes of spine rods 102 and pedicle screws 104 to be removed using the same device. It will be appreciated by those skilled in the art that changes could be made to the various exemplary embodiments described above without departing from the broad inventive concept thereof. The scope of the invention is defined by the appended claims.

## Claims

1. A spine rod cutter (20) comprising:
a helicopter socket (56) comprising a substantially cylindrical body (564) including L-shaped cut-outs (563) for engaging a spine rod; and
a hole saw (54) coaxial with and circumscribing the helicopter socket for operatively engaging the spine rod.

2. The spine rod cutter of claim 1, further comprising a driven assembly (70) operatively engaged with the hole saw and for connecting to a drive.

3. The spine rod cutter of claim 2, wherein the driven assembly comprises:
a drive connector (28); and
a thrust housing (32) for operatively engaging the hole saw.

4. The spine rod cutter of any of claims 1 to 3, further comprising a guard (62) covering the hole saw.

5. The spine rod cutter of any of claims 1 to 4, further comprising a centering mount (66) within the helicopter socket.

6. The spine rod cutter of any of claims 1 to 5, further comprising a handle (46) having a lever (48) operatively connected to the hole saw for applying an axial load to the hole saw.

7. The spine rod cutter of any of claims 1 to 6, further comprising an engagement assembly (80) releasably engaging the hole saw and helicopter socket to operate cooperatively.

8. The spine rod cutter of claim 7, further comprising:
a hole saw extension (58) extending from the hole saw; and
a helicopter socket extension (60) extending from the helicopter socket,
wherein the engagement assembly operatively connects the hole saw extension and helicopter socket extension together.

9. The spine rod cutter of claim 8, wherein the engagement assembly includes:
a knob (36) circumscribing the helicopter socket extension;
a sleeve (34) extending from the hole saw extension;
a transfer housing (35) engaged with the helicopter socket extension; and
a fastener for operatively engaging both the sleeve and transfer housing.

10. The spine rod cutter of claim 8 or claim 9, wherein the helicopter socket extension includes a colleted proximal end (603); and
wherein the driven assembly comprises:
a drive connector (28);
a thrust pin (33) housed within the drive connector operatively engaging the proximal end of the helicopter socket extension;
a thrust housing (32) for operatively engaging the hole saw; and
a thrust race (31) between the thrust housing and the helicopter socket extension.

11. The spine rod cutter of claim 10, wherein the driven assembly further comprises a sleeve lock (30) connected to the thrust pin for moving the thrust pin between first and second positions.

12. The spine rod cutter of any of claims 1 to 11, wherein the guard circumscribes the helicopter socket and the hole saw.

## Patentansprüche

1. Wirbelsäulenstabschneider (20), der Folgendes umfasst:
eine Helikopter-Buchse (56), die einen im Wesentlichen zylindrischen Körper (564) umfasst, der L-förmige Ausschnitte (563) einschließt, zum Ineingriffnehmen eines Wirbelsäulenstabs; und
eine Lochsäge (54), die mit der Helikopter-Buchse koaxial ist und diese umschreibt, zum betriebsmäßigen Ineingriffnehmen des Wirbelsäulenstabs.

2. Wirbelsäulenstabschneider nach Anspruch 1, der ferner eine angetriebene Anordnung (70) umfasst, die mit der Lochsäge betriebsmäßig im Eingriff steht, und zum Verbinden mit einem Antrieb.

3. Wirbelsäulenstabschneider nach Anspruch 2, wobei die angetriebene Anordnung Folgendes umfasst:
einen Antriebsverbinder (28); und
ein Druckgehäuse (32) zum betriebsmäßigen Ineingriffnehmen der Lochsäge.

4. Wirbelsäulenstabschneider nach einem der Ansprüche 1 bis 3, der ferner einen Schutz (62) umfasst, der die Lochsäge abdeckt.

5. Wirbelsäulenstabschneider nach einem der Ansprüche 1 bis 4, der ferner eine Zentrierhalterung (66) in der Helikopter-Buchse umfasst.

6. Wirbelsäulenstabschneider nach einem der Ansprüche 1 bis 5, der ferner einen Griff (46) umfasst, der einen Hebel (48) aufweist, der mit der Lochsäge betriebsmäßig verbunden ist, zum Anwenden einer Axiallast auf die Lochsäge.

7. Wirbelsäulenstabschneider nach einem der Ansprüche 1 bis 6, der ferner eine Eingriffsanordnung (80) umfasst, die die Lochsäge und die Helikopter-Buchse lösbar in Eingriff nimmt, um zusammenwirkend zu arbeiten.

8. Wirbelsäulenstabschneider nach Anspruch 7, der ferner Folgendes umfasst:
eine Lochsägeverlängerung (58), die sich aus der Lochsäge erstreckt; und
eine Helikopter-Buchse-Verlängerung (60), die sich aus der Helikopter-Buchse erstreckt,
wobei die Eingriffsanordnung die Lochsägeverlängerung und die Helikopter-Buchse-Verlängerung betriebsmäßig miteinander verbindet.

9. Wirbelsäulenstabschneider nach Anspruch 8, wobei die Eingriffsanordnung Folgendes umfasst:
einen Knopf (36), der die Helikopter-Buchse-Verlängerung umschreibt;
eine Hülse (34), die sich aus der Lochsägeverlängerung erstreckt;
ein Transfergehäuse (35) das mit der Helikopter-Buchse-Verlängerung im Eingriff steht; und
ein Befestigungselement zum betriebsmäßigen Ineingriffnehmen sowohl der Hülse als auch des Transfergehäuses.

10. Wirbelsäulenstabschneider nach Anspruch 8 oder Anspruch 9, wobei die Helikopter-Buchse-Verlängerung ein mit einem Spannelement versehenes proximales Ende (603) einschließt; und
wobei die angetriebene Anordnung Folgendes umfasst:
einen Antriebsverbinder (28);
einen Druckbolzen (33), der in dem Antriebsverbinder eingehaust ist und das proximale Ende der Helikopter-Buchse-Verlängerung betriebsmäßig in Eingriff nimmt;
ein Druckgehäuse (32) zum betriebsmäßigen Ineingriffnehmen der Lochsäge; und
eine Druckscheibe (31) zwischen dem Druckgehäuse und der Helikopter-Buchse-Verlängerung.

11. Wirbelsäulenstabschneider nach Anspruch 10, wobei die angetriebene Anordnung ferner eine Hülsenarretierung (30) umfasst, die mit dem Druckbolzen verbunden ist, zum Bewegen des Druckbolzen zwischen einer ersten und zweiten Position.

12. Wirbelsäulenstabschneider nach einem der Ansprüche 1 bis 11, wobei der Schutz die Helikopter-Buchse und die Lochsäge umschreibt.

## Revendications

1. Dispositif de coupe de tige rachidienne (20) comprenant :
une douille hélicoptère (56), comprenant un corps (564) sensiblement cylindrique comportant des échancrures en L (563), destinée à entrer en prise avec une tige rachidienne ; et
une scie-cloche (54), coaxiale avec la douille hélicoptère et l'entourant, destinée à entrer en prise opérante avec la tige rachidienne.

2. Dispositif de coupe de tige rachidienne selon la revendication 1, comprenant, en outre, un ensemble entraîné (70) en prise opérante avec la scie-cloche et destiné à être raccordé à un mécanisme d'entraînement.

3. Dispositif de coupe de tige rachidienne selon la revendication 2, dans lequel l'ensemble entraîné comprend :
un élément de raccordement (28) pour mécanisme d'entraînement ; et
un réceptacle de poussée (32) destiné à entrer en prise opérante avec la scie-cloche.

4. Dispositif de coupe de tige rachidienne selon l'une quelconque des revendications 1 à 3, comprenant, en outre, un élément de protection (62) couvrant la scie-cloche.

5. Dispositif de coupe de tige rachidienne selon l'une quelconque des revendications 1 à 4, comprenant, en outre, un support de centrage (66) à l'intérieur de la douille hélicoptère.

6. Dispositif de coupe de tige rachidienne selon l'une quelconque des revendications 1 à 5, comprenant, en outre, une poignée (46) comportant un levier (48) en raccordement opérant avec la scie-cloche pour appliquer une charge axiale à la scie-cloche.

7. Dispositif de coupe de tige rachidienne selon l'une quelconque des revendications 1 à 6, comprenant, en outre, un ensemble de mise en prise (80) mettant en prise de manière libérable la scie-cloche et la douille hélicoptère de façon à ce qu'elles coopèrent.

8. Dispositif de coupe de tige rachidienne selon la revendication 7, comprenant en outre :
une rallonge de scie-cloche (58) s'étendant à partir de la scie-cloche ; et
une rallonge de douille hélicoptère (60) s'étendant à partir de la douille hélicoptère,
dans lequel l'ensemble de mise en prise met la rallonge de scie-cloche et la rallonge de douille hélicoptère en raccordement opérant l'une avec l'autre.

9. Dispositif de coupe de tige rachidienne selon la revendication 8, dans lequel l'ensemble de mise en prise comprend :
une molette (36) entourant la rallonge de douille hélicoptère ;
un manchon (34) s'étendant à partir de la rallonge de scie-cloche ;
un réceptacle de transfert (35) en prise avec la rallonge de douille hélicoptère ; et
un élément de fixation destiné à entrer en prise opérante à la fois avec le manchon et avec le réceptacle de transfert.

10. Dispositif de coupe de tige rachidienne selon la revendication 8 ou la revendication 9, dans lequel la rallonge de douille hélicoptère comprend une extrémité proximale de serrage (603) ; et
dans lequel l'ensemble entraîné comprend :
un élément de raccordement (28) pour mécanisme d'entraînement ;
une broche de poussée (33) logée à l'intérieur de l'élément de raccordement pour mécanisme d'entraînement, entrant en prise opérante avec l'extrémité proximale de la rallonge de douille hélicoptère ;
un réceptacle de poussée (32) destiné à entrer en prise opérante avec la scie-cloche ; et
une goulotte de poussée (31) entre le réceptacle de poussée et la rallonge de douille hélicoptère.

11. Dispositif de coupe de tige rachidienne selon la revendication 10, dans lequel l'ensemble entraîné comprend, en outre, une bague de blocage (30) raccordée à la broche de poussée pour déplacer la broche de poussée entre des première et seconde positions.

12. Dispositif de coupe de tige rachidienne selon l'une quelconque des revendications 1 à 11, dans lequel l'élément de protection entoure la douille hélicoptère et la scie-cloche.
